# EUROPEAN PATENT APPLICATION

(11) **EP 4 691 398 A1**
(43) Date of publication of application: **11.02.2026**
(21) Application number: 25153693.4
(22) Date of filing: 24.01.2025
(51) Int. Cl.: A61B 18/12, A61B 18/14, A61B 18/00, A61B 18/16

(54) **HEMORRHOID TREATMENT SYSTEM AND ASSOCIATED METHODS**

(30) Priority: 06.08.2024 US 202418795908
(71) Applicant: MEDFINITI IP, LLC, Las Vegas, Nevada 89148 (US)
(72) Inventor: Goldman, Stephen, Palm Harbor, 34683 (US); Goeree, Michael, Tampa, 33602 (US)
(74) Representative: Fabry, Bernd

(57) **Abstract**

A hemorrhoid treatment system includes a computing device, and a handheld device coupled to the computing device. The handheld device includes a probe electrode to contact a hemorrhoid on a patient that is to be treated. A grounding pad is to be coupled to the patient. The handheld device includes a variable resistance network, and a controller to sense a feedback voltage at a current sensing resistor in response to the probe electrode contacting the hemorrhoid and to the patient being grounded via the grounding pad. A level of current being delivered to the hemorrhoid is determined based on the sensed feedback voltage. Resistance of the variable resistance network is changed to adjust the output voltage to compensate for fluctuations in body resistance of the patient so that the level of current being delivered to the hemorrhoid matches a set level of current.

## Description

### Technical Field

The present disclosure relates to the treatment of hemorrhoids, and, more particularly, to a hemorrhoid treatment system that delivers an electrical current to a hemorrhoid that is to be treated, and associated methods.

### Background

Hemorrhoids, also called piles, are swollen veins in the anus and lower rectum. As the hemorrhoidal vein becomes more dilated from the increased pressure, it may eventually form a sac-like protrusion if the condition is allowed to persist. Hemorrhoids are generally classified as being either internal or external, depending on their location relative to the dentate line.

The pain often associated with hemorrhoids may be great enough to interfere with normal patterns of defecation which in turn leads to constipation and further aggravation of the swelling in the region. In general, most symptoms associated with hemorrhoids are sufficient enough to interfere with a person's daily life causing them to require relief from a medical practitioner and/or a pharmaceutical preparation.

There is a wide range of therapies available that attempt to provide a person suffering from hemorrhoids with some level of relief. One option is medicated ointments and creams which generally relieve either the itching or inflammation, but few have been successful in reducing or completely eliminating both. Thus, the efficacy of medicated ointments and creams in relieving or curing the symptoms of hemorrhoids is uncertain.

Another option for treating hemorrhoids is surgery. Treatment via surgery ranges from minor surgery to major invasive surgery. Minor surgery may relieve symptoms through drainage of the swelling. Major invasive surgery, for example, includes the removal of extensive or severe hemorrhoids in a process known as a hemorrhoidectomy. This surgical procedure can be used on both internal and external hemorrhoids. However, a hemorrhoidectomy typically involves a long recovery period, along with the associated risks and expense of invasive surgery.

Another example for treating hemorrhoids is to deliver an electrical current to the hemorrhoids. U.S. Patent No. 9,179,966 provides a method of treating hemorrhoids that includes inputting a predetermined current time unit into a current delivering medical instrument, wherein the predetermined current time unit is based on the grade of the hemorrhoid. The hemorrhoid is contacted with a probe electrode of the current delivering medical instrument, and current is delivered to the hemorrhoid. A delivered current time unit is determined. The delivered current time unit includes a product comprised of current delivered multiplied by time. A visual and/or auditory indication is provided with the medical instrument when the predetermined current time unit amount is achieved.

Nonetheless, even in view of the above, there is still a need to improve how hemorrhoids are treated.

### Summary

A hemorrhoid treatment system includes a computing device and a handheld device. The handheld device has a cable coupled to the computing device, with the cable comprising an inline connector junction. A probe electrode may have a first end coupled to the handheld device, and a second end to contact a hemorrhoid on a patient that is to be treated. A grounding pad may be coupled to the patient. A grounding pad cable may be coupled between the grounding pad and the inline connector junction.

The handheld device may include a voltage output configured to deliver an output voltage to the probe electrode, a variable resistance network coupled to the voltage output, a current sensing resistor configured to provide a feedback voltage, and a controller coupled to the current sensing resistor and the variable resistance network.

The controller may be configured to sense the feedback voltage at the current sensing resistor in response to the second end of the probe electrode contacting the hemorrhoid and to the patient being grounded via the grounding pad, and determine a level of current being delivered to the hemorrhoid based on the sensed feedback voltage. The controller may be configured to change resistance of the variable resistance network to adjust the output voltage to compensate for fluctuations in body resistance of the patient so that the level of current being delivered to the hemorrhoid matches a set level of current.

The controller may periodically sense the feedback voltage at the current sensing resistor. The variable resistance network may include a plurality of resistive inputs, with each resistive input corresponding to a different resistive value. The controller may include a sensing and adjusting algorithm to periodically sense the feedback voltage, and to adjust the resistance of the variable resistance network by selecting one of the plurality of resistive inputs so that the level of current being delivered to the hemorrhoid matches the set level of current.

The cable may be configured to provide power and ground from the computing device to the handheld device. The grounding pad cable may be coupled to ground in the inline connector junction.

The computing device may include operational treatment software configured to perform handshaking with the handheld device in order for the handheld device to operate. The operational treatment software may also be configured to generate a treatment summary report in response to the handheld device being powered off after treatment of the hemorrhoid.

The treatment summary report may be stored in a medical records database. The treatment summary report may include identification of the patient, a duration of how long current was delivered to the hemorrhoid, and the level of the current delivered to the hemorrhoid during the duration.

The handheld device may include a timer display, and may be configured to operate in a manual mode or a timer mode. The manual mode may correspond to the timer display counting up from zero during treatment of the hemorrhoid. The timer mode may correspond to the timer display counting down from a selected time set on the timer display.

The handheld device may include a current bar graph display to display the current level being delivered to the hemorrhoid.

The probe electrode may be bifurcated as a pair of spaced apart elongated electrode members each with an electrode tip for contacting the hemorrhoid.

A spacer may slidably engage the elongated electrode members in order to maintain a desired spacing between the electrode tips.

A base overmold may be positioned over a portion of the elongated electrode members, and an open extension may extend from the base between inward facing surfaces of the elongated electrode members while exposing outward facing surfaces of the elongated electrode members.

The probe electrode may be a continuous length of conductive material folded in half to define a closed end and an open end. The closed end may be configured as a spring for press-fitting into the handheld device. The open end may be configured as a pair of spaced apart electrode tips for contacting the hemorrhoid.

The handheld device may include a housing enclosing the voltage output, the variable resistance network, the current sensing resistor and the controller. A plastic insert may be secured within the housing and has an opening extending therethrough. An electrical plug may have a receptacle end and a threaded end, with the receptacle end positioned within the opening in the plastic insert and configured to receive the first end of the probe electrode. The threaded end may extend through a backside of the plastic insert. An electrical lug may be positioned over the threaded end of the electrical plug for contacting the voltage output. A nut may be secured to the threaded end of the electrical plug for securing the electrical plug within the plastic insert.

The handheld device may include at least one light directed to the second end of the probe electrode that contacts the hemorrhoid.

Another aspect is directed to a handheld device that may include a housing, an electrical plug carried by the housing and having a receptacle end configured to receive a first end of a probe electrode, with a second end of the probe electrode configured to contact a hemorrhoid on a patient that is to be treated. A voltage output may be carried by the housing and configured to deliver an output voltage to the probe electrode via the electrical plug. A variable resistance network may be carried by the housing and coupled to the voltage output. A current sensing resistor may be carried by the housing and configured to provide a feedback voltage. A controller may be carried by the housing and coupled to the current sensing resistor and the variable resistance network. The controller may be configured to sense the feedback voltage at the current sensing resistor in response to the second end of the probe electrode contacting the hemorrhoid and to the patient being grounded, and determine a level of current being delivered to the hemorrhoid based on the sensed feedback voltage. The controller may change resistance of the variable resistance network to adjust the output voltage to compensate for fluctuations in body resistance of the patient so that the level of current being delivered to the hemorrhoid matches a set level of current.

Yet another aspect is directed to a method for using a handheld device to treat a hemorrhoid on a patient as described above. The method may include grounding the patient via a grounding pad, providing power to the handheld device, and positioning the handheld device so that a second end of the probe electrode contacts the hemorrhoid on the patient. The method may include operating the controller to sense the feedback voltage at the current sensing resistor in response to the second end of the probe electrode contacting a hemorrhoid on a patient that is to be treated and to the patient being grounded, and determine a level of current being delivered to the hemorrhoid based on the sensed feedback voltage. The controller may change resistance of the variable resistance network to adjust the output voltage to compensate for fluctuations in body resistance of the patient so that the level of current being delivered to the hemorrhoid matches a set level of current.

### Brief Description of the Drawings

FIG. 1 is a block diagram of a hemorrhoid treatment system in which various aspects of the disclosure may be implemented.
FIG. 2 is a view of the handheld device, probe electrode, grounding pad and grounding pad cable illustrated in FIG. 1.
FIG. 3 is a back side perspective view of the handheld device and probe electrode illustrated in FIG. 1 without the cable.
FIG. 4 is a front side perspective view of the handheld device and probe electrode illustrated in FIG. 1 without the cable.
FIG. 5A is a side view of a single length of electrically conductive material bent in half to form part of the probe electrode illustrated in FIG. 1.
FIG. 5B is a side view of the electrically conductive material illustrated in FIG. 5A with shrink tubing.
FIG. 5C is a back side perspective view of the electrically conductive material with shrink tubing illustrated in FIG. 5B with a base overmold.
FIG. 6A is a back side perspective view of the probe electrode illustrated in FIG. 5C with a slidable spacer.
FIG. 6B is a front view of the slidable spacer illustrated in FIG. 6A.
FIGS. 7A-7C are different views of another embodiment of the probe electrode illustrated in FIG. 5C with a base overmold closed extension.
FIGS. 8A-8C are different views of another embodiment of the probe electrode illustrated in FIG. 5C with a base overmold open extension.
FIG. 9 is a partially exploded view of the handheld device configured to receive the probe electrode illustrated in FIG. 1.
FIG. 10 is a partial view of the probe electrode coupled to the handheld device illustrated in FIG. 9.

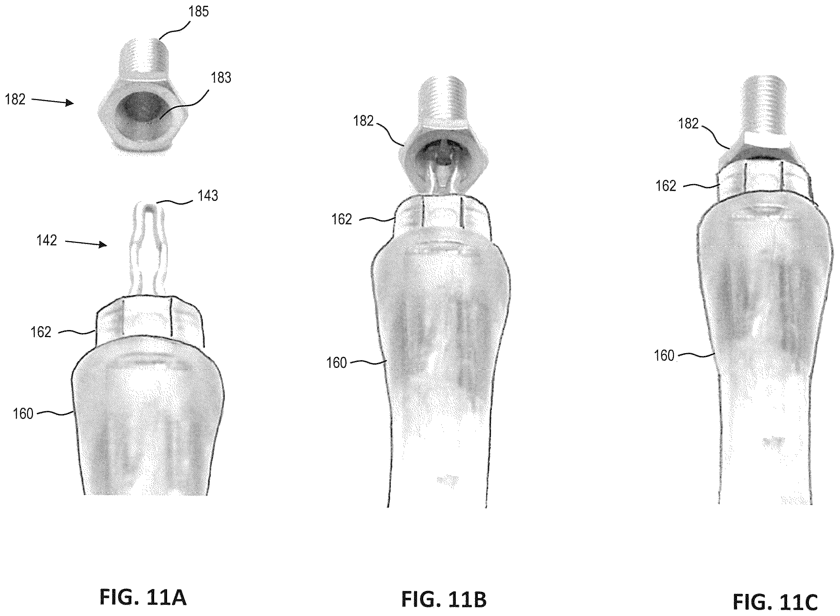

FIGS. 11A-11C are sequence views of the closed end of the probe electrode being inserted into the electrical plug within the handheld device illustrated in FIG. 10.

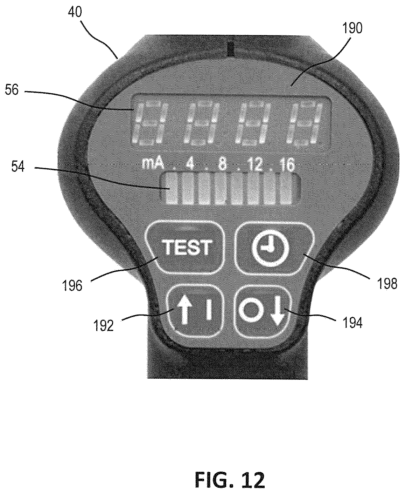

FIG. 12 is a view of a user interface membrane placed on the user interface area of the handheld device illustrated in FIG. 3.

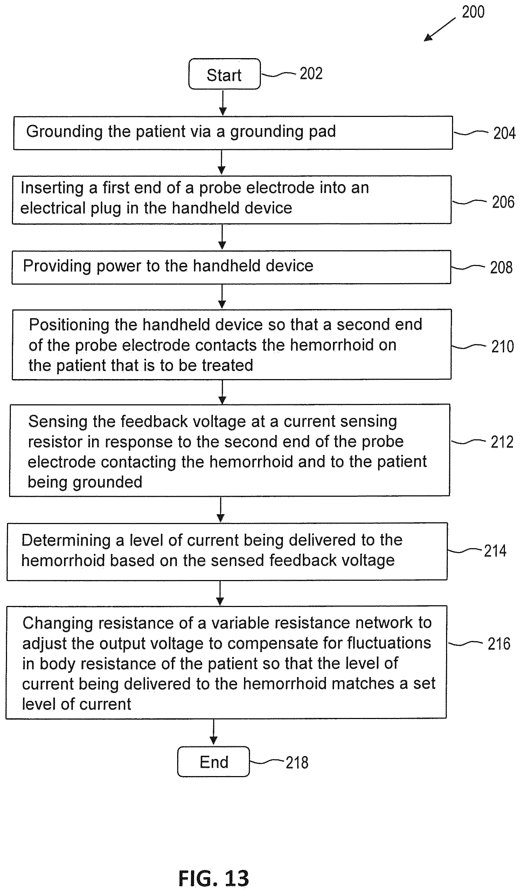

FIG. 13 is a flowchart for using the handheld device illustrated in FIG. 1 to treat a hemorrhoid on a patient.

### Detailed Description

The present description is made with reference to the accompanying drawings, in which exemplary embodiments are shown. However, many different embodiments may be used, and thus the description should not be construed as limited to the particular embodiments set forth herein. Rather, these embodiments are provided so that this disclosure will be thorough and complete. Like numbers refer to like elements throughout.

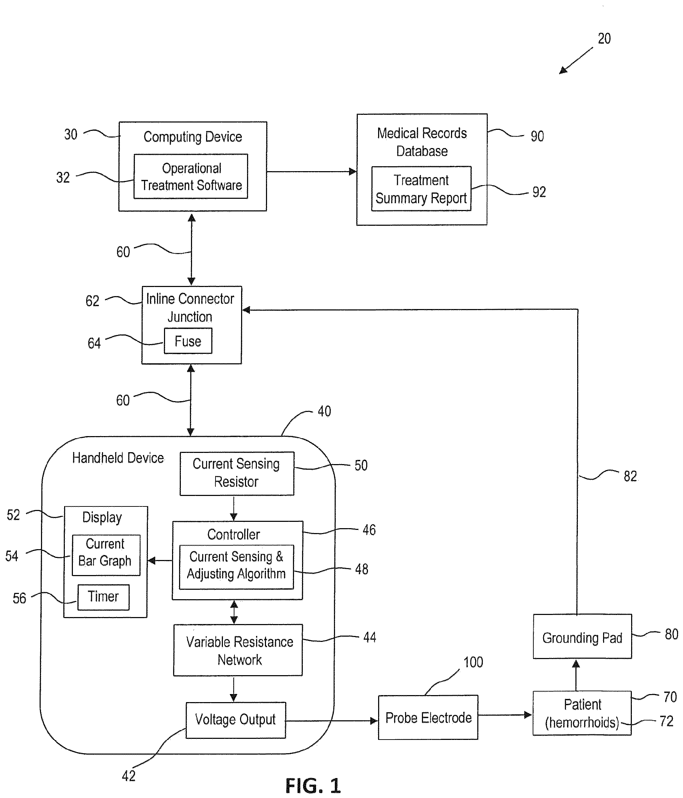

Referring initially to FIG. 1, a hemorrhoid treatment system **20** for a patient **70** having hemorrhoids **72** that are to be treated will be discussed. The hemorrhoid treatment system **20** utilizes direct current electrotherapy to reduce and eliminate symptoms of hemorrhoid disease, which is estimated to affect up to one-third of the population in the United States.

As will be described in detail below, the hemorrhoid treatment system **20** is advantageously configured to automatically deliver a steady level of current to the hemorrhoid **72** by using feedback to adjust an output voltage to compensate for fluctuations in body resistance of the patient **70.** As noted in the above Background section, U.S. Patent No. 9,179,966 disclosed the use of electrical current to treat hemorrhoids. A limitation of the '966 patent is that manual intervention was required to adjust for variations in the level of current being delivered to the hemorrhoid due to fluctuations in body resistance of the patient.

The hemorrhoid treatment system **20** includes a computing device **30,** and a handheld device **40** coupled to the computing device **30** via a cable **60.** The computing device **30** is configured to provide power and ground to the handheld device **40** over the cable **60,** as well as to exchange data signals with the handheld device **40.**

The computing device **30** may be a laptop or desktop computing device, for example. The computing device **30** is configured to execute operational treatment software **32** for interfacing with the handheld device **40** over the cable **60.** One of the functions of the operational treatment software **32** is to perform handshaking with the handheld device **40.** Handshaking is required to authorize operation of the handheld device **40.** After authorization, operation of the handheld device **40** may be self-sustained to operate on its own.

The operational treatment software **32** is also configured to generate a treatment summary report **92** after the patient **70** has been treated, which may be used for billing purposes. The treatment summary report **92** may include identification of the patient, a duration of how long current was delivered to the hemorrhoid **72,** and the level of the current delivered to the hemorrhoid **72** during the duration. The patient identification may be, for example, by name, social security number, or an assigned patient number. The treatment summary report **92** may be stored in a medical records database **90.**

A probe electrode **100** has a first end coupled to the handheld device **40,** and a second end configured to contact or engage the hemorrhoid **72** that is to be treated. A grounding pad **80** is to be coupled to the patient **70.** The cable **60** includes an inline connector junction **62.** A grounding pad cable **82** is coupled between the grounding pad **80** and the inline connector junction **62,** where the inline connector junction **62** provides a ground to the grounding pad **80.** The inline connector junction **62** may include a fuse **64** in series between the grounding pad **80** and a grounding wire within the cable **60** to limit a maximum amount of current that may be delivered to the hemorrhoid **72.**

The handheld device **40** includes a voltage output **42** configured to deliver an output voltage to the probe electrode **100,** a variable resistance network **44** coupled to the voltage output **42,** and a current sensing resistor **50** configured to provide a feedback voltage. The variable resistance network **44** has a plurality of selectable resistance configurations.

A controller **46** is coupled to the current sensing resistor **50** and to the variable resistance network **44.** The controller **46** is configured to operate a current sensing and adjusting algorithm **48** to periodically sense the feedback voltage at the current sensing resistor **50** in response to the second end of the probe electrode **100** contacting the hemorrhoid **72,** and to the patient **70** being grounded via the grounding pad **80.** The patient **70** acts as a resistive load to form an electrical circuit between the probe electrode **100** and the grounding pad **80.**

A level of current being delivered to the hemorrhoid **72** is determined by the current sensing and adjusting algorithm **48** using Ohm's law. Ohm's law is a formula used to calculate the relationship between voltage, current and resistance in an electrical circuit.

In response to the probe electrode **100** contacting the hemorrhoid **72** and the patient **70** being grounded via the grounding pad **80,** an output voltage is applied to the probe electrode **100.** The current flowing through the hemorrhoid **72** ramps up from 0 to 16 milliamps (mA) within about 10 seconds. The electrical current is a steady, low-level current that causes smooth muscle contraction and thrombosis resulting in permanent ligation of the hemorrhoid's feeder blood vessels. The use of 16 mA is one example current level that may be used for treating hemorrhoids **72.** A 16 mA current may be applied for a time determined by the hemorrhoid's grade for a 90-95% cure rate. The doctor or gastroenterologist will determine the hemorrhoid grade and thus choose the procedural time needed. Since the vast majority of patients are unable to tolerate 16 mA of current, anesthesia is typically used.

As noted above, the level of current being delivered to the hemorrhoid **72** is determined by the controller **46** based on the feedback voltage being measured at the current sensing resistor **50.** The feedback voltage is periodically measured. As an example, this measurement may be in the milliseconds range, such as every 32 milliseconds.

The handheld device **40** includes a display **52** coupled to the controller **46.** The display 52 provides a current bar graph **54** and a timer **56.** The current bar graph **54** displays the level of current being delivered to the hemorrhoid **72.** When current is being delivered to the hemorrhoid **72,** the timer **56** changes time. The handheld device **40** may operate in a manual mode or a timer mode. In the manual mode, the timer **56** counts up from zero. In the timer moder, the timer **56** counts down to zero from a time selected by the doctor.

In response to the level of current being delivered to the hemorrhoid **72,** the controller **46** changes resistance of the variable resistance network **44** to adjust the output voltage to compensate for fluctuations in body resistance of the patient **70** so that the level of current being delivered to the hemorrhoid **72** matches a desired or set level of current. The desired level of current is 16 mA in this example embodiment, and may be considered a default value for the level of current to be applied to the hemorrhoid **72** without additional input from the doctor.

The controller **46** may have a plurality of output ports that interfaces with a corresponding plurality of inputs to the resistive network **44,** where each input corresponds to a different resistance configuration. The controller **46 46** changes resistance of the variable resistance network **44** by selecting the appropriate output port that connects to a desired resistance configuration within the resistive network **44.**

Fluctuations in body resistance, for example, may be due to movement by the patient **70** with respect to the grounding pad **80.** In addition, saline is a conductive solution that is typically used to apply the grounding pad **80** to the patient **70.** If the saline starts to dry out, then this may lead to fluctuations in body resistance of the patient **70.** The handheld device **40** advantageously treats the hemorrhoid **72** by delivering the desired level of current to the hemorrhoid **72** within a very tight tolerance. The tolerance may be within a range of 0.5 - 1.5 percent, for example.

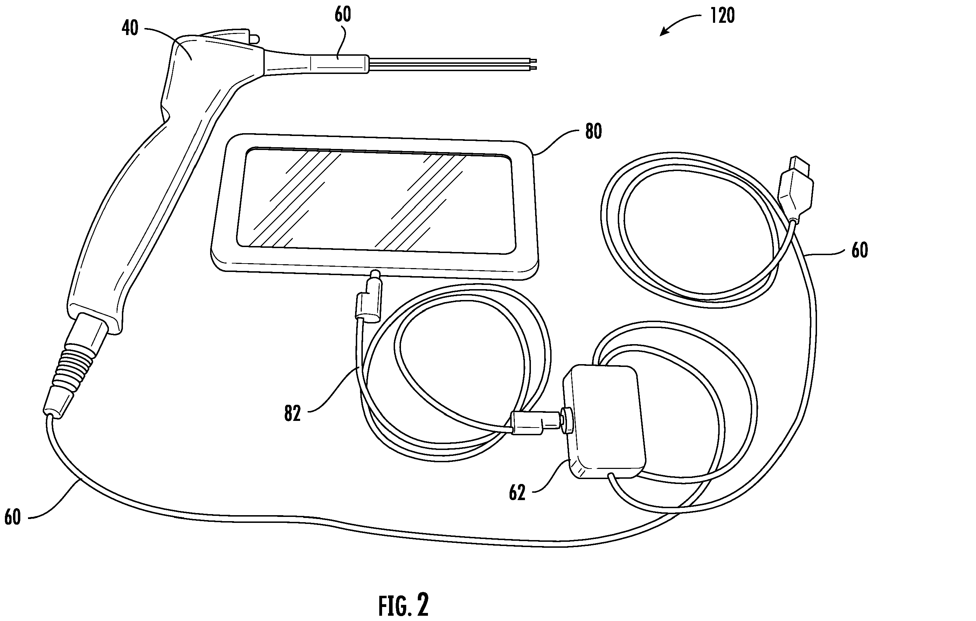

A view **120** of the handheld device **40** with cable **60** and inline connector junction **62,** probe electrode **100,** grounding pad **80** and grounding pad cable **82** is provided in FIG. 2. Since the grounding pad **80** is disposable, it is removably coupled to one end of the ground pad cable **82.** The other end of the grounding pad cable **82** is removably coupled to the inline connector junction **62.** One end of the cable **60** is coupled within the handheld device **40,** with the free end of the cable **60** being removably coupled to the computing device **30.** The cable **60** may be configured as a USB (universal serial bus) cable. A USB cable allows data exchanges between the computing device **30** and the handheld device **40,** and provides power and ground to the handheld device **40** as well as providing ground to the ground pad **80** via the inline connector junction **62.**

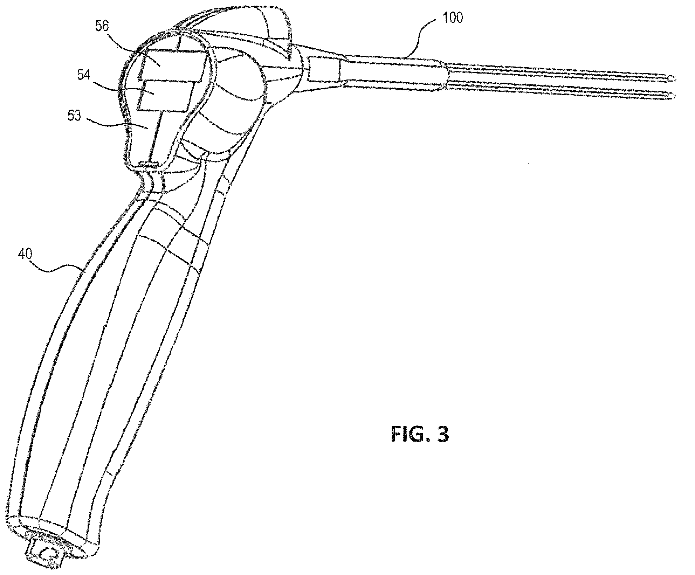

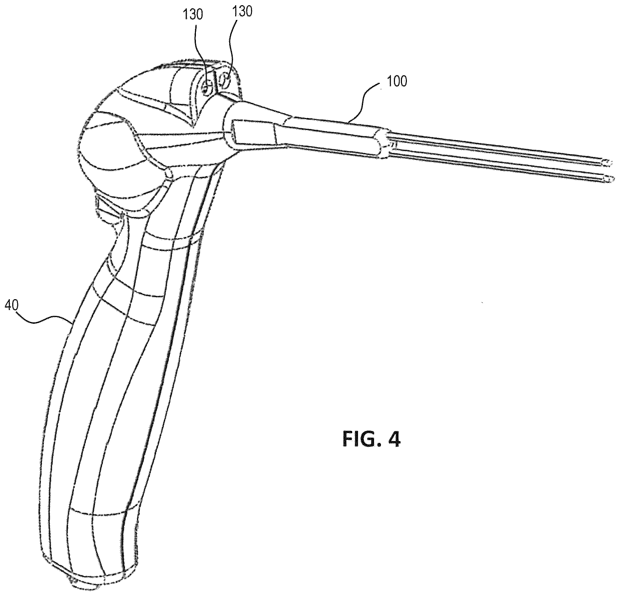

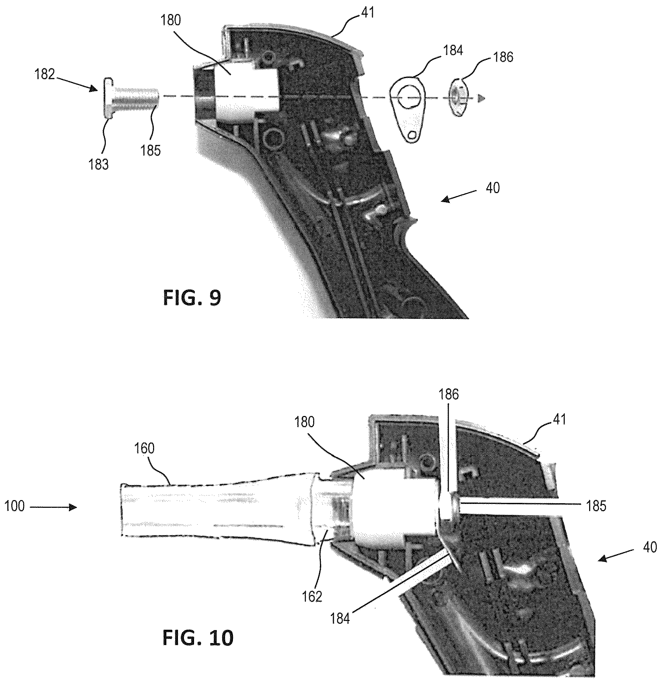

Referring now to FIGS. 3 and 4, a back side perspective view and a front perspective view of the handheld device **40** with the probe electrode **100** are provided. The handheld device **40** includes a user interface area **53** that is visible to the doctor from the backside of the handheld device **40.** The display area **53** includes the current bar graph **54** and the timer **56.** As will be discussed in greater detail below with reference to FIG. 9, a user interface membrane **190** is placed over the display area **53** which provides user interface buttons for operating the handheld device **40.**

The handheld device **40** further includes at least one light **130** directed to the second end of the probe electrode **100** that contacts the hemorrhoid. This results in safer and more accurate procedures. In the illustrated embodiment, a pair of lights **130** are provided. The lights may be configured as light emitting diodes (LEDs).

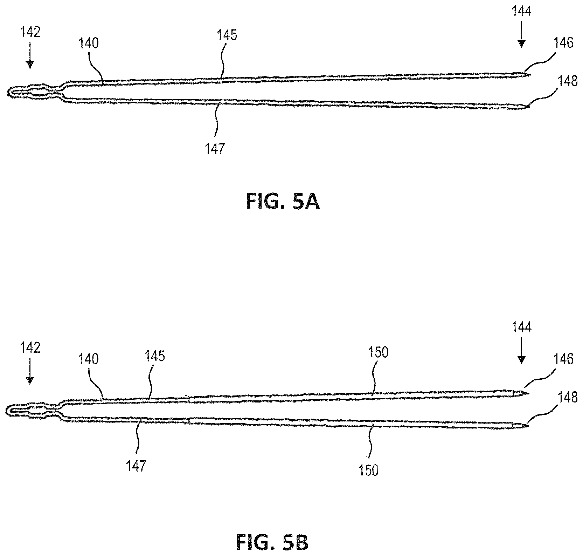

Referring now to FIGS. 5A-5C, the probe electrode **100** will be discussed in greater detail. The probe electrode **100** includes a single length of electrically conductive material **140** that is folded in half, with a bend region forming a closed end **142** of the probe electrode **100,** as shown in FIG. 5A. Extending from the bend region is a first elongated electrode member **145** that includes a first electrode tip **146,** and a second elongated electrode member **147** that includes a second electrode tip **146.** The first and second electrode tips **146, 148** form an open end of the probe electrode **100.** Each electrode tip **146, 148** is preferably beveled to form a point.

The closed end **142** corresponds to the first end of the probe electrode **100,** and the open end **144** corresponds to the second end of the probe electrode **100.** In other words, the probe electrode **100** is bifurcated as a pair of spaced apart elongated electrode members **145, 147** each with an electrode tip **146, 148** for contacting the hemorrhoid **72.**

The electrically conductive material may be a metal wire, such as stainless steel, copper, etc. The metal wire is heat annealed at approximately its center point, and is then bent about 180 degrees to form the bend region. This allows for the electrode tips **146, 148** to be divergent from one another.

Shrink tubing **150** is placed over the first elongated electrode member **145** and over the second elongated electrode member **147** while leaving the respective electrode tips **146, 148** exposed, as shown in FIG. 5B. The single length of electrically conductive material **140** with the shrink tubing **150** thereon is placed into an oven at a temperature and time sufficient to shrink the tubing **150** around the respective first and second elongated electrode members **145, 147.** Typically, 15 minutes @ 300-500 degrees F is sufficient for this purpose.

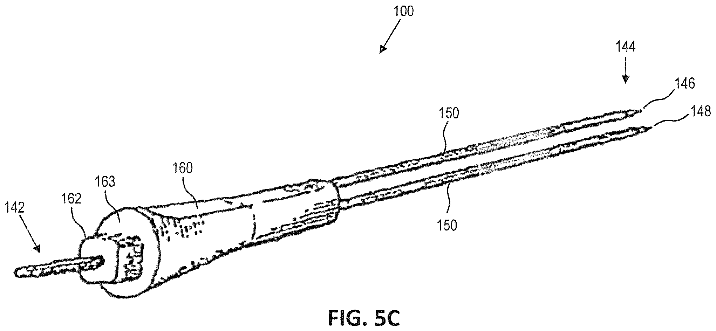

After shrinking the tubing **150,** a base overmold **160** is associated with the first and second elongated electrode members **145, 147,** as shown in FIG. 5C. The base overmold **160** provides a grip area for holding the probe electrode **100** while interfacing (i.e., coupling/decoupling) with the handheld device **40.** In one embodiment this is achieved through plastic mold injection. The molds are designed to seal around a portion of the first and second elongated electrode members **145, 147** including a portion of the shrink tubing **150.**

In one embodiment, the plastic used for the base overmold **160** is C10 made by Adept Polymers, Ltd, and the injection is conducted @ 11,000 PSI, @ 350-400 degrees F. The plastic injection forms a 4-sided keying block **162** with a physical stop **163.** This is intended for mating with the handheld device **40.** The 4-sided keying block **162** allows for the positioning of the probe electrode **100** in two vertical positions and two horizontal positions.

As noted above, the elongated electrode members **145, 147** and the electrode tips **146, 148** are divergent from one another. A default spacing of the electrode tips **146, 148** is obtained when the base overmold **160** is formed. For a default spacing of 5 mm, for example, the electrode tips **146, 148** are held at this spacing while the base overmold **160** is formed. After the base overmold **160** has been formed, the default spacing of the electrode tips **146, 148** is set. The default spacing of the electrode tips **146, 148** is not limited to 5 mm. The default spacing may vary within a range of 5 mm - 12 mm, for example.

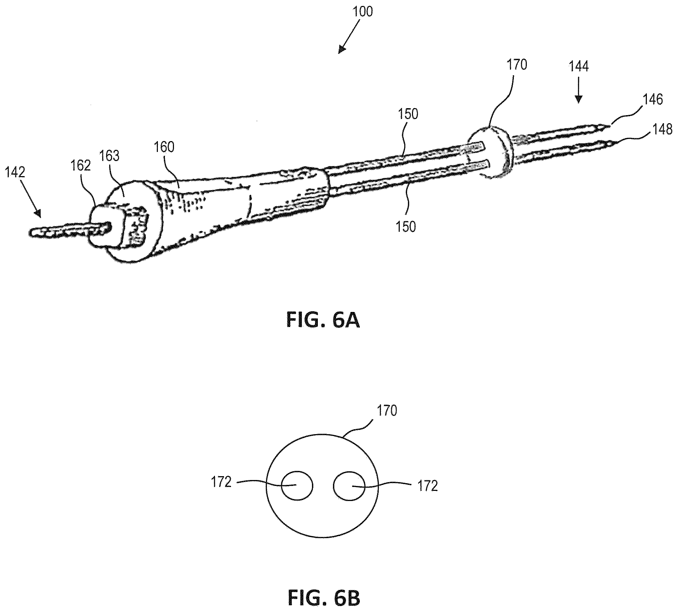

To ensure that spacing of the electrode tips **146, 148** is at the default range, or if the doctor would like to change the default spacing, a spacer **170** may be used, as shown in FIG. 6AC. The spacer **170** includes a pair of spaced apart openings **172,** as shown in FIG. 6B, for slidably engaging the elongated electrode members **145, 147** of the probe electrode **100.**

The spacer 170 may be formed out of an elastic, flexible and moldable material, such as polyurethane or rubber, for example. Prior to treating the hemorrhoid **72,** the doctor slides the spacer **170** over the electrode tips **146 148** and over the elongated electrode members **145, 147** until the desired spacing is achieved. The shrink tubing **150** placed over the elongated electrode members **145, 147** may include one or more markers calibrated to assist in positioning of the spacer **170** to achieve the desired spacing.

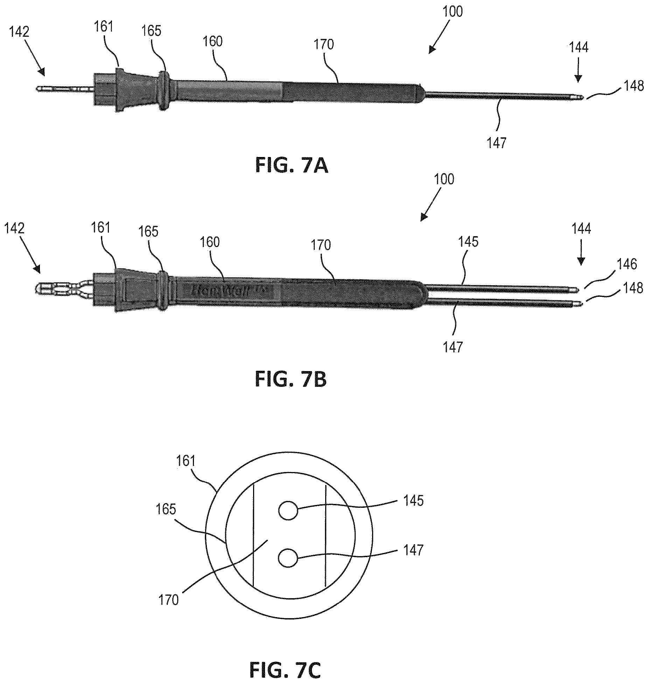

Referring now to FIGS. 7A-7C, the base overmold **160** may be formed with a closed extension **170.** The closed extension **170** is a continuation of the base overmold **160** and partially extends over the elongated electrode members **145, 147.** The closed extension **170** helps to insure that the electrode tips **146 148** are held at the default spacing.

The base overmold **160** may be formed with base sections **161, 165** such that a portion of the base overmold **160** therebetween is tapered from base section **161** towards base section **165.** From base section **165** towards the electrode tips **146, 148,** a profile of the base overmold **160** matches a profile of the closed extension **170.**

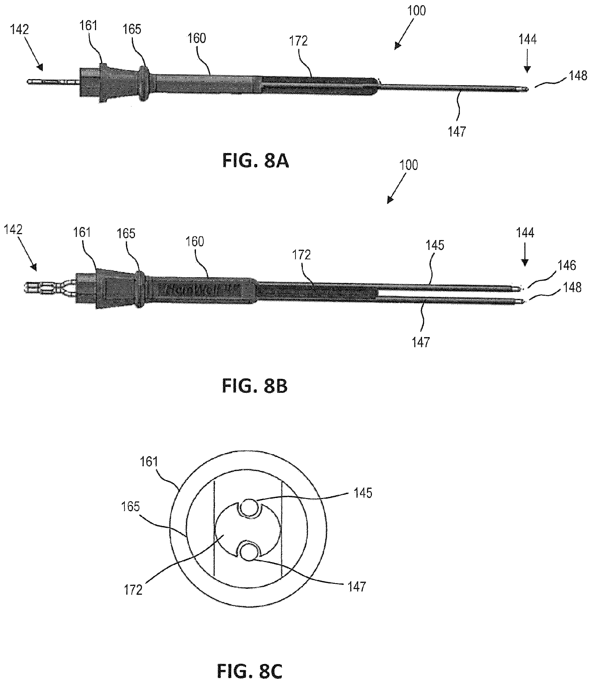

Referring now to FIGS. 8A-8C, the base overmold **160** may be formed with an open extension **172.** The open extension **172** is a continuation of the base overmold **160** but exposes the outward facing surfaces of the elongated electrode members **145, 147.** The open extension **172** is formed between the inward facing surfaces of the elongated electrode members **145, 147.** A profile of the open extension **172** is circular and includes a pair of openings to receive the inward facing surfaces of the elongated electrode members **145, 147** while the outward facing surfaces are exposed.

Although not shown, the final packaging of the probe electrode **100** includes a plastic guard to prevent accidental puncture and a gas permeable sleeve for sterilization. Due to the plastic being water-soluble, gas sterilization or E-Beam is used. Steam sterilization is prohibited.

Referring now to FIG. 6, a partially exploded view of the handheld device **40** configured to receive the probe electrode **100** will be discussed. The handheld device **40** includes a housing **41** that defines a shape of the handheld device **40.** The housing **41** may be formed by joining together two housing halves. One of the housing halves **41** is removed to illustrate where the first end of the probe electrode **100** is to be coupled to the handheld device **40.**

A plastic insert **180** is secured within the housing **41.** The housing **41** includes a tapered extension and slotted fins within the housing. The plastic insert **180** is held in place by being positioned between the tapered extension and the slotted fins. The plastic insert **180** has an opening extending therethrough, and an electrical plug **182** is to be positioned within the opening. The electrical plug **182** has a receptacle end **183** and a threaded end **185.** The receptacle end **183** is configured to receive the closed end **142** of the probe electrode **100.** The threaded end **183** is to extend through a backside of the plastic insert **180.**

An electrical lug **184** is to be positioned over the threaded end **185** of the electrical plug **182.** An electrical wire will extend from the electrical lug **184** to the voltage output 42. The electrical wire provides a path for voltage from the voltage output **42** to the electrical plug **182** which is then transferred to the probe electrode **100.** A nut **186** may be used to secure the electrical plug **182** and the electrical lug **184** to the plastic insert **180.** The closed end **142** of the probe electrode **100** is inserted in the receptacle end **183** of the electrical plug **182,** as shown in FIG. 7.

Referring now to FIGS. 8A-8C, a sequence of steps for inserting the closed end **142** of the probe electrode **100** into the receptacle end **183** of the electrical plug **182** will be discussed. As noted above, the electrical plug **182** is position within the opening in the plastic insert **180,** and is secured to the plastic holder **180** using a nut **186** positioned on the threaded end **185** of the electrical plug **182**

The closed end **142** of the probe electrode **100** is configured as a spring, as shown in FIG. 8A, for press-fitting into the receptacle end **183** of the electrical plug **182.** As the tip **143** of the closed end **142** bottoms out within the receptacle end **183,** the closed end **142** starts to expand outwards, as shown in FIG. 8B. With the closed end **142** fully inserted into the receptacle end **183** of the electrical plug **182,** as shown in FIG. 8C, expansion of the closed end firmly holds the probe electrode **100** in place.

Although the plastic insert **180** is not shown, an outermost portion of the opening extending therethrough is shaped to receive the keying block **162** of the base overmold **160** of the probe electrode **100.** Prior to insertion of the probe electrode **100,** the keying block **162** may be rotated so that electrode tips **146, 148** are oriented in either a vertical or horizontal position with respect to how the electrode tips **146, 148** will be contacting the hemorrhoid **72.**

Referring now to FIG. 9, the user interface membrane **190** positioned over the display area **53** of the handheld device **40** will be discussed. As noted above, the display **52** includes a current bar graph **54** and a timer **56.** The user interface membrane **190** includes an opening for the current bar graph **54** and an opening for the timer **56.**

The user interface membrane **190** is coupled to the controller **46** and provides user interface buttons for operating the handheld device **40.** The user interface membrane **190** includes an on and up arrow button **192,** an off and down arrow button **194,** a self-test button **196** and a timer button **198.**

A self-test of the handheld device **40** is to be run before each use, unless this function has been disabled by the doctor via software. To initiate the self-test, the doctor engages the grounding pad **80** with the probe electrode **100** and presses the self-test button **196.**

During the self-test, each digit on the timer **56** is tested to make sure all the segments are working correctly. Then as part of the self-test, the handheld device **40** sweeps from delivering 0 to 16 milliamps (mA) and back to 0. As the current sweeps for 0 to 16 mA, the current bar graph **54** displays a bar at every 2 mA increment. When 16 mA is being delivered by the handheld device **40,** then 8 bars appear. As the current sweeps back to 0, a bar will be removed every 2 mA decrement.

The self-test also requires the doctor to press the up arrow button **192** and the down arrow button **194** to make sure these buttons are working. If everything is working correctly, then "pass" is displayed on the timer **56.** In order to remove display of "pass" the doctor presses the timer button **198.** This causes all zeros to appear on the timer **56.**

The doctor now has the option to operate the handheld device **40** in a manual mode or a timer mode. In the manual mode, the doctor engages the hemorrhoid **72** and then presses the up arrow button **192** for 3 seconds. The 3 second delay before current is delivered to the hemorrhoid **72** helps to prevent the handheld device **40** from accidentally delivering current when it is not intended to do so.

As current is being delivered to the hemorrhoid **72,** the timer **56** counts up from zero. The handheld device **40** is configured to deliver 16 mA as a default current level setting. If the doctor wants less current to be delivered, then the doctor presses the down arrow button **194** until the desired number of bars appear on the timer **56,** where each bar represents 2 mA.

If current is not being delivered to the hemorrhoid **72,** then the timer **56** stops counting until current is actually being delivered again. For instance, the doctor may move the probe electrode **100** so that contact is no longer being made with the hemorrhoid **72.** Once contact with the hemorrhoid **72** is again made by the probe electrode **100,** then the timer **56** will display the bars indicating that current is being delivered by the handheld device **40.** After an appropriate amount of time has passed in treating the hemorrhoid **72,** the doctor presses the down arrow button **194.**

For the doctor to operate the handheld device **40** in the timer mode, the doctor presses the timer button **198.** This causes the timer **56** to flash or blink. Each press of the timer button **198** adds 30 seconds to the timer **56.** After the desired amount of time has been entered, the doctor presses the timer button **56** again which causes the set time on the timer **56** to stop flashing. Next, the doctor presses the up arrow button **192** for 3 second. As current is being delivered to the hemorrhoid **72,** the timer **56** counts down from the set time to zero.

Another aspect is directed to a method for using the handheld device **40** as discussed above to treat a hemorrhoid **72** on a patient **70.** Referring now to the flowchart **200** in FIG. 10, from the start (Block **202**), the method includes grounding the patient **70** via the grounding pad **80** at Block **204.** The first end of the probe electrode **100** is inserted into the electrical plug **182** at Block **206.** Power is provided by the computing device **30** to the handheld device **40** over cable **60** at Block **208.** The handheld device **40** is then positioned at Block **210** so that a second end of the probe electrode **100** contacts the hemorrhoid **72.**

The controller **46** within the handheld device **40** is operated to sense the feedback voltage at the current sensing resistor **50** at Block **212** in response to the second end of the probe electrode **100** contacting the hemorrhoid **72** and to the patient **70** being grounded. A level of current being delivered to the hemorrhoid **72** based on the sensed feedback voltage is determined at Block **214.**

The resistance of the variable resistance network **44** is changed at Block **216** to adjust the output voltage to compensate for fluctuations in body resistance of the patient **70** so that the level of current being delivered to the hemorrhoid matches a set level of current. The method ends at Block **218.**

Many modifications and other embodiments will come to the mind of one skilled in the art having the benefit of the teachings presented in the foregoing descriptions and the associated drawings. Therefore, it is understood that the foregoing is not to be limited to the example embodiments, and that modifications and other embodiments are intended to be included within the scope of the appended claims.

## Claims

1. A hemorrhoid treatment system comprising:
a computing device;
a handheld device comprising a cable coupled to the computing device, with the cable comprising an inline connector junction;
a probe electrode having a first end coupled to the handheld device, and a second end configured to contact a hemorrhoid on a patient that is to be treated;
a grounding pad configured to be coupled to the patient; and
a grounding pad cable coupled between the grounding pad and the inline connector junction;
the handheld device comprising:
a voltage output configured to deliver an output voltage to the probe electrode,
a variable resistance network coupled to the voltage output,
a current sensing resistor configured to provide a feedback voltage, and
a controller coupled to the current sensing resistor and the variable resistance network, and configured to perform the following:
sense the feedback voltage at the current sensing resistor in response to the second end of the probe electrode contacting the hemorrhoid and to the patient being grounded via the grounding pad,
determine a level of current being delivered to the hemorrhoid based on the sensed feedback voltage, and
change resistance of the variable resistance network to adjust the output voltage to compensate for fluctuations in body resistance of the patient so that the level of current being delivered to the hemorrhoid matches a set level of current.

2. The hemorrhoid treatment system according to claim 1
wherein the controller periodically senses the feedback voltage at the current sensing resistor.

3. The hemorrhoid treatment system according to claim 1
wherein the variable resistance network comprises a plurality of resistive inputs, with each resistive input corresponding to a different resistive value; and wherein the controller comprises a sensing and adjusting algorithm to periodically sense the feedback voltage, and to adjust the resistance of the variable resistance network by selecting one of the plurality of resistive inputs so that the level of current being delivered to the hemorrhoid matches the set level of current.

4. The hemorrhoid treatment system according to claim 1
wherein the cable is configured to provide power and ground from the computing device to the handheld device, and wherein the grounding pad cable is coupled to ground in the inline connector junction.

5. The hemorrhoid treatment system according to claim 1
wherein the computing device comprises operational treatment software configured to perform handshaking with the handheld device in order for the handheld device to operate.

6. The hemorrhoid treatment system according to claim 1
wherein the computing device comprises operational treatment software configured to generate a treatment summary report in response to the handheld device being powered off after treatment of the hemorrhoid, with the treatment summary report to be stored in a medical records database.

7. The hemorrhoid treatment system according to claim 6
wherein the treatment summary report includes identification of the patient, a duration of how long current was delivered to the hemorrhoid, and the level of the current delivered to the hemorrhoid during the duration.

8. The hemorrhoid treatment system according to claim 1
wherein the handheld device comprises:
a timer display and is configured to operate in a manual mode or a timer mode, with the manual mode corresponding to the timer display counting up from zero during treatment of the hemorrhoid, and with the timer mode corresponding to the timer display counting down from a selected time set on the timer display; and
a current bar graph display to display the current level being delivered to the hemorrhoid.

9. The hemorrhoid treatment system according to claim 1
wherein the probe electrode is bifurcated as a pair of spaced apart elongated electrode members each with an electrode tip for contacting the hemorrhoid.

10. The hemorrhoid treatment system according to claim 9 comprising a spacer for slidably engaging the elongated electrode members in order to maintain a desired spacing between the electrode tips.

11. The hemorrhoid treatment system according to claim 9 comprising a base overmold positioned over a portion of the elongated electrode members, and an open extension extending from the base between inward facing surfaces of the elongated electrode members while exposing outward facing surfaces of the elongated electrode members.

12. The hemorrhoid treatment system according to claim 1
wherein the probe electrode is a continuous length of conductive material folded in half to define a closed end and an open end, with the closed end configured as a spring for press-fitting into the handheld device, and with the open end configured as a pair of spaced apart electrode tips for contacting the hemorrhoid.

13. The hemorrhoid treatment system according to claim 1
wherein the handheld device comprises:
a housing enclosing the voltage output, the variable resistance network, the current sensing resistor and the controller;
a plastic insert secured within the housing and having an opening extending therethrough;
an electrical plug having a receptacle end and a threaded end, with the receptacle end positioned within the opening in the plastic insert and configured to receive the first end of the probe electrode, and with the threaded end extending through a backside of the plastic insert;
an electrical lug positioned over the threaded end of the electrical plug for contacting the voltage output; and
a nut secured to the threaded end of the electrical plug for securing the electrical plug within the plastic insert.

14. The hemorrhoid treatment system according to claim 1
wherein the handheld device comprises at least one light directed to the second end of the probe electrode that contacts the hemorrhoid.

15. A method for using a handheld device to treat a hemorrhoid on a patient, the handheld device comprising a housing; an electrical plug carried by the housing and having a receptacle end configured to receive a first end of a probe electrode; a voltage output carried by the housing to deliver an output voltage to the probe electrode via the electrical plug; a variable resistance network carried by the housing and coupled to the voltage output; a current sensing resistor carried by the housing to provide a feedback voltage; and a controller carried by the housing and coupled to the current sensing resistor and the variable resistance network, the method comprising:
grounding the patient via a grounding pad;
providing power to the handheld device;
positioning the handheld device so that a second end of the probe electrode contacts the hemorrhoid on the patient; and
operating the controller to perform the following:
sense the feedback voltage at the current sensing resistor in response to the second end of the probe electrode contacting a hemorrhoid on a patient that is to be treated and to the patient being grounded,
determine a level of current being delivered to the hemorrhoid based on the sensed feedback voltage, and
change resistance of the variable resistance network to adjust the output voltage to compensate for fluctuations in body resistance of the patient so that the level of current being delivered to the hemorrhoid matches a set level of current.
